# EUROPEAN PATENT APPLICATION

(11) **EP 4 438 028 A1**
(43) Date of publication of application: **02.10.2024**
(21) Application number: 22898859.8
(22) Date of filing: 25.10.2022
(51) Int. Cl.: A61K 8/02, A61K 8/25, A61K 8/99, A61Q 19/00

(54) **POROUS SILICA IMPREGNATED WITH EPIDERMIDIBACTERIUM KERATINI EXTRACT AND USE THEREOF FOR IMPROVING SKIN CONDITION**

(30) Priority: 26.11.2021 KR 20210166108
(71) Applicant: Cosmax, Inc., Hwaseong-si, Gyeonggi-do 18622 (KR)
(72) Inventor: HEO, Young Mok, Seongnam-si, Gyeonggi-do 13376 (KR); LEE, Dong Geol, Hwaseong-si, Gyeonggi-do 18496 (KR); KIM, Min Ji, Seongnam-si, Gyeonggi-do 13486 (KR); KIM, Mi Sun, Seongnam-si, Gyeonggi-do 13486 (KR); KANG, Seung Hyun, Seoul 06285 (KR); PARK, Myeong Sam, Seoul 04987 (KR)
(74) Representative: Lavoix
(86) International application number: PCT/KR2022/016368
(87) International publication number: WO 2023/096177

(57) **Abstract**

Provided are porous silica impregnated with an extract of an *Epidermidibacterium keratini* strain, a cosmetic composition including the same, and a method of producing the cosmetic composition. Porous silica according to one aspect is impregnated with an *E. keratini* strain extract, which is a water-insoluble component, and thus can improve the negative feeling of use characteristic of the water-insoluble component and be formulated stably. In addition, the porous silica has an excellent skin improvement effect, and thus can be used as a cosmetic composition.

## Description

### Technical Field

This application claims priority to Korean Patent Application No. 10-2021-0166108, filed on November 26, 2021, the entire contents of which are hereby incorporated by reference.

The present disclosure relates to porous silica impregnated with a microbial extract, a cosmetic composition including the same, and use thereof for improving skin condition.

### Background Art

Culture solutions, fermented products, extracts, and the like of microorganisms have recently been widely used as effective ingredients in the cosmetics market. Among them, in the case of water-insoluble extracts or water-insoluble substances derived from microorganisms, there is a problem wherein they are highly reactive, have low stability, and often have negative feelings such as slipperiness or a sticky feeling when applied to the skin. Therefore, it has been difficult to apply water-insoluble components derived from microorganisms as cosmetics.

Accordingly, the present inventors studied to overcome the problems of the prior art and confirmed that in the case of porous silica impregnated with an *Epidermidibacterium keratini* extract, it can not only solve the above problems but also has excellent skin functionality, and the present disclosure was completed.

### Disclosure

### Technical Problem

One aspect is to provide porous silica impregnated with an extract of an *Epidermidibacterium keratini* strain.

Another aspect is to provide a cosmetic composition including porous silica according to one aspect.

Another aspect is to provide use of a cosmetic composition including porous silica according to one aspect.

Another aspect is to provide a method of producing a cosmetic composition according to one aspect.

### Technical Solution

One aspect provides porous silica impregnated with an extract of an *Epidermidibacterium keratini* strain.

The type of strain is not limited as long as it belongs to the species *E. keratini.* For example, the strain may be an *E. keratini* strain described in Korean Patent Publication KR 10-1910791, but is not limited thereto. The strain may be a strain deposited under deposit number KCCM11843P. The strain deposited under above deposit number may be an *E. keratini* EPI-7 strain.

The strain may include 16S rRNA having a sequence identity of about 95 % or more, about 96 % or more, about 97 % or more, about 98 % or more, about 99 % or more, about 99.5 % or more, or about 99.9 % or more with 16S rRNA sequence of the E. *keratini* EPI-7 strain (deposit number: KCCM11843P). The strain may include the 16S rRNA sequence of the *E. keratini* EPI-7 strain (deposit number: KCCM11843P).

The term "sequence identity" refers to the degree of identity of amino acid residues or bases between sequences after aligning both sequences to achieve maximum matching in a specific comparison area. Sequence identity can be found according to methods known in the art. The percentage of sequence identity can be determined using known sequence comparison programs, examples of which may include BLASTN(NCBI), CLC Main Workbench (CLC bio), MegAlignTM(DNASTAR Inc), etc.

The extract may be a lysis solution, a culture solution, a fermented product, or an extract of bacterial cells of the *E. keratini* strain.

The term "lysis solution" may be used interchangeably with "lysate," and may refer to a product obtained by fracturing the cell wall of a strain by chemical or physical force. The lysis solution may include the lysis solution itself, its concentrate, or its lyophilisate.

The term "culture solution" may be used interchangeably with "culture supernatant," "conditioned culture," or "conditioned medium," and may refer to the entire medium including the strain, its metabolites, extra nutrients, etc. obtained by culturing the strain for a certain period of time in a medium capable of supplying nutrients to enable the strain to grow and survive in vitro. Additionally, the culture solution may refer to a culture solution from which the bacterial cells are removed from a bacterial culture solution obtained by culturing a strain. On the other hand, the liquid from which the bacterial cells have been removed from the culture solution is also called "supernatant", and the supernatant can be obtained by leaving the culture solution still for a certain period of time and taking only the liquid of the upper layer excluding the part that has settled in the lower layer, removing the bacterial cells through filtration, or removing the lower precipitation by centrifuging the culture solution and taking only the upper liquid.

Culture media and culture conditions for culturing the *E. keratini* strain can be utilized by being appropriately selected or modified by those skilled in the art. For example, the culture solution may be obtained by culturing in a medium (e.g., R2A medium or TSB medium) at any temperature above 10°C or below 40°C for a certain period of time, for example, 4 hours to 120 hours.

The culture solution may include the culture solution itself obtained by culturing the strain, a concentrate thereof or a lyophilisate thereof, or a culture supernatant obtained by removing the strain from the culture solution, a concentrate thereof or a lyophilisate thereof.

The term "fermented product" may refer to any substance obtained through a metabolic process in which chemical changes occur in organic matter by the action of microorganisms. The fermented product may refer to a fermented broth obtained through fermentation metabolism of the strain or components included therein. The fermented product may include the fermented product itself, a concentrate thereof or a lyophilisate thereof.

The term "bacterial cells" refers to the strain itself of the present disclosure, and includes the strain itself selected by isolation from a sample, etc., or a strain isolated from the culture solution by culturing the strain. The bacterial cells can be obtained by centrifuging the culture solution and taking the part that has settled in the lower layer, or since they settle in the lower layer of the culture solution by gravity, they can be obtained by leaving them still for a certain period of time and then removing the upper liquid.

The term "extract" includes all substances obtained by extracting components of a strain, regardless of the extraction method, extraction solvent, extraction conditions, extracted components, or form of the extract, and also includes substances obtained by processing or treating by other methods after extracting the components of the strain. For example, the processing or treatment may include dilution, concentration, drying, purification, fractionation, filtration, fermentation, enzyme treatment, etc. Therefore, the extract may include an extract, a diluted or concentrated solution of the extract, a dried product obtained by drying the extract, or a crude or purified product thereof, or a fraction obtained by fractionation thereof.

The extraction method is not limited to a specific extraction method, but may be, for example, stirred extraction.

The extraction solvent may be oil. By using oil as the extraction solvent, water-insoluble components can be extracted from the strain.

In one embodiment, the oil may be one or more selected from the group consisting of rice bran oil, medium chain triglycerides (MCT) oil, soybean oil, sunflower seed oil, jojoba oil, mineral oil, squalane, caprylic/capric triglyceride, cetyl ethylhexanoate, dodecane, isododecane, and hydrogenated polydecene, but is not limited thereto. The MCT oil is also called liquid coconut oil. The oil may be rice bran oil.

The extract may be an oil extract of a strain culture solution. Oil as the extraction solvent may be used in an amount of 0.01 times to 100 times, 0.01 times to 50 times, 0.01 times to 20 times, 0.01 times to 10 times, 0.01 times to 5 times, 0.1 times to 100 times, 0.1 times to 50 times, 0.1 times to 20 times, 0.1 times to 10 times, 0.1 times to 5 times, 0.1 times to 1 times, 0.5 times to 100 times, 0.5 times to 50 times, 0.5 times to 20 times, 0.5 times to 10 times, 0.5 times to 5 times, or 0.5 times to 1 time, based on the total volume of the culture solution, but is not limited thereto.

The extraction conditions refer to conditions for extracting the components of the strain, such as extraction time, extraction temperature, etc. The extraction time may be 30 minutes to 120 hours, 30 minutes to 100 hours, 30 minutes to 80 hours, 30 minutes to 60 hours, 2 hours to 120 hours, 2 hours to 100 hours, 2 hours to 80 hours, 2 hours to 60 hours, 10 hours to 120 hours, 10 hours to 100 hours, 10 hours to 80 hours, or 10 hours to 60 hours, but can be appropriately selected depending on other conditions such as extraction solvent, extraction temperature, etc. The extraction temperature may be 10 °C to 100 °C, 10 °C to 80 °C, 20 °C to 100 °C, 20 °C to 80 °C, 20 °C to 60 °C, 20 °C to 40 °C, or room temperature, but can be appropriately selected depending on other conditions such as extraction solvent, extraction time, etc.

The strain, its lysis solution, culture solution, fermentation product, and extract thereof may be biologically pure. The strain may be obtained by pure culture.

The porous silica may be characterized by small particle size, large specific surface area, microporosity, etc. The porous silica may be produced using an appropriate method known to those skilled in the art, and those commercially available may also be used.

The porous silica may be silicon dioxide. The porous silica may consist of silicon dioxide.

The porous silica may have a particle diameter of 0.1 µm to 100 µm, 0.1 µm to 50 µm, 0.1 µm to 20 µm, 0.2 µm to 100 µm, 0.2 µm to 50 µm, 0.2 µm to 20 µm, 0.5 µm to 100 µm, 0.5 µm to 50 µm, 0.5 µm to 20 µm, 1 µm to 100 µm, 1 µm to 50 µm, 1 µm to 20 µm, 3 µm to 100 µm, 3 µm to 50 µm, or 3 µm to 20 µm, but is not limited thereto.

The porous silica may have a specific surface area of 100 m²/g to 1000 m²/g, 200 m²/g to 1000 m²/g, or 500 m²/g to 1000 m²/g, but is not limited thereto.

The porous silica may have a pore volume of 0.5 cm³/g to 5 cm³/g, 0.5 cm³/g to 3 cm³/g, 0.5 cm³/g to 2 cm³/g, 1 cm³/g to 5 cm³/g, 1 cm³/g to 3 cm³/g, or 1 cm³/g to 2 cm³/g, but is not limited thereto.

The porous silica may have a pore size of 1 nm to 100 nm, 1 nm to 50 nm, 1 nm to 30 nm, 1 nm to 20 nm, 2 nm to 100 nm, 2 nm to 50 nm, 2 nm to 30 nm, or 2 nm to 20 nm, but is not limited thereto.

The porous silica may have a homogeneous particle size. The porous silica may have excellent pore uniformity.

The surface of the porous silica may be hydrophobically coated. The porous silica may include an organic coating that imparts hydrophobicity to the surface. The organic coating may be applied to the inorganic coating, if present, or directly to the silica.

The hydrophobically coated porous silica may effectively penetrate the water-insoluble components into the inner and outer surfaces of the porous silica, thereby impregnating it with the water-insoluble components. When porous silica is coated with a water-soluble polymer, it may not be possible to impregnate it with water-insoluble components.

The hydrophobic coating agent for the porous silica may be, for example, silicone, silane, metal soap, titanate, organic wax, and mixtures thereof. The hydrophobic coating agent may alternatively include fatty acids, for example, fatty acids including 10 to 20 carbon atoms, such as lauric acid, stearic acid, isostearic acid, and salts of these fatty acids. The fatty acids may be isopropyl titanium triisostearate.

The silicone hydrophobic coating agent for the porous silica may be methicone, dimethicone, or copolymers or mixtures thereof. The silicone also may be organosilicon compounds, for example, dimethylpolysiloxane with a backbone of repeating -Me₂SiO units ("Me" is methyl, CH3), methyl hydrogen polysiloxane with a backbone of repeating -MeHSiO units, and alkoxysilane of the formula RnOSiH₍₄₋ₙ₎ ("R" is alkyl and "n" is an integer of 1, 2 or 3).

The silane hydrophobic coating agent for the porous silica may be alkoxysilane. The alkoxysilane may be triethoxycaprylylsilane, or perfluoroalkylethyl triethoxysilane having a straight-chain or branched C3-C12 alkyl group.

The metal soap hydrophobic coating agent for the porous silica may be metal myristate, metal stearate, metal palmitate, metal laurate or other fatty acid derivatives known to those skilled in the art. The metal may be, for example, magnesium or aluminum.

The organic wax hydrophobic coating agent for the porous silica may be a synthetic wax such as polyethylene or a natural wax such as carnauba wax.

In one embodiment, the surface of the porous silica may be coated with methicone, dimethicone, or copolymers or mixtures thereof. The surface of the porous silica may be coated with methicone.

When the silica particles are coated or "surface treated," the material forming the coating or treatment may be in an amount of about 0.1 wt% to about 35 wt%, about 0.1 wt% to about 30 wt%, about 0.1 wt% to about 20 wt%, about 0.1 wt% to about 10 wt%, about 0.1 wt% to about 5 wt%, or about 0.1 wt% to about 1 wt% of the silica, but is not limited thereto.

The *E. keratini* extract may be impregnated into the porous silica at an oil absorption of 10 mL/100 g to 500 mL/100 g, 10 mL/100 g to 400 mL/100 g, 10 mL/100 g to 300 mL/100 g, 20 mL/100 g to 500 mL/100 g, 20 mL/100 g to 400 mL/100 g, 20 mL/100 g to 300 mL/100 g, 50 mL/100 g to 500 mL/100 g, 50 mL/100 g to 400 mL /100 g, 50 mL/100 g to 300 mL/100 g, or 60 mL/100 g to 270 mL/100 g, but is not limited thereto.

The hydrophobically coated silica particles may effectively and stably store active ingredients with high skin absorption, such as essential fatty acids and lipids, due to their microporous structure. When water-insoluble components are impregnated into the inside of porous silica particles, they have less influence on other compositions during storage and thus can be formulated stably. In addition, it can provide a refreshing and light feeling by eliminating the sticky or slippery sensation of water-insoluble components.

In one Example, it was confirmed that compared to the *E. keratini* oil extract, the porous silica impregnated with it eliminates the sticky or slippery sensation of water-insoluble components and thus provides a refreshing and light feeling, thereby having excellent feeling of use on the skin such as freshness, moisture, adhesion, etc.

Additionally, the porous silica may have additional effects due to the characteristics of the silica particles themselves. For example, silica particles may easily disintegrate when applied to the skin, filling fine wrinkles and pores and providing a moisturized and adhesive skin feeling. In addition, due to the low refractive index of the silica particles themselves, they may provide a translucent appearance while covering curves. Additionally, when the water-insoluble components impregnated into porous silica are absorbed into the skin, the porous silica regains its oil absorption power, and thus can absorb excessively produced sebum and prevent shine and stickiness.

Another aspect provides a cosmetic composition comprising the porous silica impregnated with the extract of the *E. keratini* strain according to one aspect.

The cosmetic composition has a skin improvement effect. Specifically, the skin improvement may include improving skin condition, improving skin beauty, and preventing, improving, or treating skin diseases. The skin improvement may be any one or more of preventing skin aging, ameliorating skin wrinkles, strengthening skin barrier, moisturizing skin, enhancing skin elasticity, ameliorating dead skin cells, improving skin gloss, improving skin transparency, and improving skin tone. Therefore, the cosmetic composition may be for preventing skin aging, ameliorating skin wrinkles, strengthening skin barrier, moisturizing skin, enhancing skin elasticity, ameliorating dead skin cells, improving skin gloss, improving skin transparency, or improving skin tone.

The term "skin aging" refers to both tangible and intangible changes that occur in the skin with age, such as the phenomenon of thinning of the epidermis thickness, reduction of the number of cells or blood vessels in the dermis, DNA damage repair ability, cell replacement cycle, wound recovery, skin barrier function, epidermal moisture retention, sweat secretion, sebum secretion, vitamin D production, physical damage defense, chemical removal ability, immune response, sensory function, and temperature regulation.

The composition may be for anti-aging or preventing skin aging caused by extrinsic or endogenous factors. The exogenous factors refer to various external factors, such as ultraviolet rays (light), and the endogenous factors are also called chronological factors and mainly refer to factors that occur due to the passage of time. In other words, skin aging includes specifically not only symptoms of premature aging induced by external stimuli such as ultraviolet rays, pollution, cigarette smoke, and chemicals, but also a natural aging phenomenon that occurs as the proliferation of skin cells decreases with age, and it is a concept that includes all of wrinkles, loss of elasticity, skin sagging, dryness, etc. In addition, wrinkles include stimulation caused by changes in internal and external factors that change the components constituting skin tissue, causing wrinkles.

The aging may be photoaging. The term "photoaging" is a phenomenon caused by external environmental factors, the most representative factor being ultraviolet rays. Ultraviolet rays result in damage to biological components such as activation of proteolytic enzymes, chain cutting of matrix proteins, and abnormal cross-linking, and repetition of this mechanism causes skin aging that is evident even in appearance. Therefore, the composition may be for preventing or improving photoaging caused by ultraviolet B (UVB).

The term "ameliorating skin wrinkles" is interpreted to mean not only the action of reducing the number or depth of wrinkles on the skin, but also include inhibiting skin aging through wrinkle amelioration.

The term "strengthening skin barrier" may refer to any action that enhances the function of the skin barrier, which is located on the outermost layer of the skin and prevents moisture and nutrition loss.

The term "moisturizing skin" may refer to any action that maintains skin moisture or prevents moisture loss. The skin moisturizing effect can help ameliorate skin wrinkles and increase skin elasticity.

The term "enhancing skin elasticity" may refer to any action that increases skin elasticity which has been reduced due to causes such as aging or prevents a decrease in skin elasticity.

The term "dead skin cells" refers to foreign substances that occur when old cells die and fall off in the epidermal layer of the skin or the like.

The term "ameliorating dead skin cells" is used interchangeably with "skin exfoliation" and may refer to removing or reducing dead skin cells.

The term "improving skin gloss" may refer to any action that increases the gloss of the skin or restores the decreased gloss. For example, the skin gloss can be measured using a skin gloss value evaluation device such as a Glossymeter.

The term "improving skin transparency" may refer to any action that increases the transparency of the skin or restores the decreased transparency. For example, the skin transparency may be measured using a skin transparency evaluation device such as a Translucency meter.

The term "improving skin tone" may refer to any action that increases skin brightness or brightens the darkened skin tone. For example, the skin tone can be measured using a skin brightness evaluation device such as a Chromameter.

The term "skin disease" may be a disease caused by skin aging or damage to the skin barrier function. The term "prevention" includes inhibiting the occurrence of a disease. The term "treatment" includes inhibiting, alleviating, or eliminating the development of a disease. The term "improvement" may refer to any action that at least reduces parameters associated with the alleviation or treatment of a condition, for example the severity of symptoms.

The composition according to one aspect may increase collagen biosynthesis or decrease collagen-degrading enzyme biosynthesis. For example, the composition may increase the expression of COL1A1 or decrease the expression of MMP-1. Therefore, the composition may have anti-aging and wrinkle- ameliorating effects on the skin.

The composition according to one aspect may increase hyaluronic acid synthesis or increase filaggrin synthesis. For example, the composition may increase the expression of HAS and filaggrin. Additionally, the composition may decrease transepidermal water loss or increase skin moisture content. Therefore, the composition may have effects of strengthening skin barrier and moisturizing skin.

The composition according to one aspect was confirmed, after use, to increase epidermal elasticity as a result of evaluating epidermal elasticity by using a Cutometer. Therefore, the composition may have an effect of enhancing skin elasticity.

The composition according to one aspect was confirmed, after use, to reduce a skin desquamation index as a result of desquamation index evaluation using Corneofix and Visioscan. Therefore, the composition may have an effect of ameliorating dead skin cells.

The composition according to one aspect was confirmed, after use, to increase a skin gloss index as a result of skin gloss index evaluation using a Glossymeter. Therefore, the composition may have an effect of improving skin gloss.

The composition according to one aspect was confirmed, after use, to reduce the rate of decrease in an amount of transmitted light as a result of skin transparency evaluation using a Translucency meter. Therefore, the composition may have an effect of improving skin transparency.

The composition according to one aspect was confirmed, after use, to increase skin brightness as a result of skin brightness evaluation using a Chromameter. Therefore, the composition may have an effect of improving skin tone.

The composition may include 0.001 wt% to 90 wt%, 0.001 wt% to 80 wt%, 0.001 wt% to 70 wt%, 0.001 wt% to 60 wt%, 0.001 wt% to 50 wt%, 0.001 wt% to 40 wt%, 0.001 wt% to 30 wt%, 0.001 wt% to 20 wt%, 0.001 wt% to 10 wt%, 0.01 wt% to 50 wt%, 0.01 wt% to 40 wt%, 0.01 wt% to 30 wt%, 0.01 wt% to 20 wt%, 0.01 wt% to 10 wt%, 0.01 wt% to 5 wt%, 0.01 wt% to 3 wt%, or 0.01 wt% to 1 wt% of the porous silica according to one aspect, based on the total weight of the composition.

The term "including as an active ingredient" means that the porous silica of the present specification is added to an extent that can produce the effects mentioned above. Additionally, this may include formulation in various forms by adding various ingredients as sub-ingredients for drug delivery, stabilization, etc.

The cosmetic composition may be produced in any commonly produced formulation. For example, the cosmetic composition may have cosmetic formulations of lotion, cream, essence, cleansing foam, cleansing water, pack, ampoule, body lotion, body oil, body gel, shampoo, rinse, hair conditioner, hair gel, foundation, lipstick, mascara, makeup base or skin-adhesive types.

In addition to the composition as active ingredients, ingredients included in the cosmetic composition may include ingredients commonly used in cosmetic compositions, for example including conventional auxiliaries and carriers such as preservatives, antioxidants, stabilizers, solubilizers, vitamins, pigments, and fragrances. The cosmetic composition may include an effective amount of additives sufficient to reduce deterioration. The additives may be, for example, binders, but are not limited thereto.

Alternatively, the composition may be a composition for external skin preparations.

The external skin preparations may be creams, gels, ointments, skin emulsifiers, skin suspensions, transdermal delivery patches, drug-containing bandages, lotions, or a combination thereof. The external skin preparations may be appropriately formulated with ingredients commonly used in external skin preparations such as cosmetics and medicines, for example aqueous ingredients, oil-based ingredients, powder ingredients, alcohols, moisturizers, thickeners, ultraviolet absorbers, whitening agents, preservatives, antioxidants, surfactants, fragrances, colorants, various skin nutrients, or a combination thereof, if necessary. The external skin preparations may be appropriately formulated with metal sequestrants such as disodium edetate, trisodium edetate, sodium citrate, sodium polyphosphate, sodium metaphosphate, and gluconic acid, caffeine, tannin, belafamil, licorice extract, glablidin, hot water extract of the fruit of calin, various herbal medicines, drugs such as tocopherol acetate, glycyrrhizinic acid, tranexamic acid, and derivatives or salts thereof, vitamin C, magnesium ascorbate phosphate, ascorbate glucoside, arbutin, kojic acid, and saccharides such as glucose, fructose, and trehalose.

Another aspect provides a method of producing the cosmetic composition according to one aspect.

The method includes producing an extract of an *E. keratini* strain by using oil as an extraction solvent;
impregnating the extract into porous silica; and
producing a cosmetic composition comprising the porous silica impregnated with the extract.

Details regarding the extraction solvent, strain, extract, porous silica, and cosmetic composition are as described above.

The extract of the *E. keratini* strain may be an extract of a culture solution of the strain.

The impregnation may be used without limitation as long as it can impregnate porous silica with the extract.

The porous silica may be hydrophobically coated.

Redundant content is omitted in consideration of the complexity of the present specification, and terms not otherwise defined in the present specification have meanings commonly used in the technical field to which the present disclosure pertains.

### Advantageous Effects

The porous silica according to one aspect is impregnated with the *Epidermidibacterium keratini* strain extract, which is a water-insoluble component, and thus may improve the negative feeling of use characteristic of the water-insoluble component and be formulated stably. In addition, the porous silica has the effects of preventing skin aging, ameliorating wrinkles, strengthening skin barrier, moisturizing, enhancing skin elasticity, ameliorating dead skin cells, improving gloss, improving transparency, and improving skin tone, and thus may be used as a cosmetic composition.

### Description of Drawings

FIG. 1 is results showing the relative expression level of collagen synthase (COL1A1) in fibroblasts after addition of porous silica of Example 2 or porous silica of a control group.
FIG. 2 is results showing the relative expression level of collagen degrading enzyme (MMP-1) in fibroblasts after addition of porous silica of Example 2 or porous silica of a control group.
FIG. 3 is results showing the relative expression level of hyaluronic acid synthase (HAS3) in keratinocytes after addition of porous silica of Example 2 or porous silica of a control group.
FIG. 4 is results showing the relative expression level of filaggrin in keratinocytes after addition of porous silica of Example 2 or porous silica of a control group.
FIG. 5 is results of evaluating transepidermal water loss (TEWL) after using porous silica of Example 2.
FIG. 6 is results of evaluating skin moisture content after using porous silica of Example 2.
FIG. 7 is results of evaluating epidermal elasticity after using porous silica of Example 2.
FIG. 8 is results of evaluating a skin desquamation index after using porous silica of Example 2.
FIG. 9 is results of evaluating a skin gloss index after using porous silica of Example 2.
FIG. 10 is results of evaluating skin transparency after using porous silica of Example 2.
FIG. 11 is results of evaluating skin brightness after using porous silica of Example 2.

### Mode for Invention

Hereinafter, the present disclosure will be described in more detail through examples. However, these examples are for illustrative purposes only and the scope of the present disclosure is not limited to these examples.

### Example 1. Production of oil extract of Epidermidibacterium keratini strain

To produce an extract of an *E. keratini* strain, oil was used as an extraction solvent.

Specifically, after culturing the *E. keratini* strain in TSB medium, rice bran oil in half volume of the culture solution was added and stirred vigorously. After leaving the mixture at room temperature for 24 hours, the rice bran oil layer was recovered to obtain a rice bran oil extract of the *E. keratini* strain.

### Example 2. Production of porous silica impregnated with E. keratini strain extract

The rice bran oil extract of the *E. keratini* strain obtained in Example 1 was impregnated into porous silica. Methicone-coated silicon dioxide was used as the porous silica. The extract was impregnated into porous silica at an oil absorption of 100 mL/100 g. The obtained porous silica impregnated with the *E. keratini* extract was used in the following experimental examples.

Meanwhile, in the following experimental examples, the same porous silica as in Example 2 was used as a control, except that it was not impregnated with the E. *keratini* extract.

### Preparation Example 1. Production of cosmetic compositions

A cosmetic composition in a cushion formulation including the porous silica impregnated with the *E. keratini* extract of Example 2 was produced as an example. The ingredients and contents (wt%) of the cosmetic composition are shown in Table 1 below.

**[Table 1]**

| **Ingredient** | **Content (%, w/w)** |
|---|---|
| Porous silica impregnated with *E. keratini* extract of Example 2 | 1.000000000 |
| *Centella asiatica* extract | 30.251485000 |
| Titanium dioxide (Cl 77891) | 13.683181000 |
| Cyclopentasiloxane | 8.300000000 |
| Ethylhexyl methoxycinnamate | 6.700000000 |
| Diphenylsiloxy phenyl trimethicone | 4.000000000 |
| Ethylhexyl salicylate | 4.000000000 |
| Propylene glycol dibenzoate | 3.500000000 |
| Trimethylsiloxysilicate | 3.000000000 |
| Lauryl PEG-9 polydimethylsiloxyethyl dimethicone | 2.999400000 |
| Glycerin | 2.345544000 |
| Diisostearyl malate | 2.000000000 |
| Isododecane | 2.000000000 |
| Niacinamide | 2.000000000 |
| Butylene glycol | 1.700100000 |
| 1,2-hexanediol | 1.510090000 |
| Purified water | 1.503155203 |
| Synthetic fluorophlogopite | 1.283500000 |
| Acrylate/stearyl acrylate/dimethicone methacrylate copolymer | 1.000000000 |
| Cetyl PEG/PPG-10/1 Dimethicone | 1.000000000 |
| Disteadimonium hectorite | 0.900000000 |
| Yellow iron oxide (Cl 77492) | 0.783020000 |
| Magnesium sulfate | 0.700000000 |
| VP/Hexadecene copolymer | 0.500000000 |
| Bismuth Oxychloride (Cl 77163) | 0.350000000 |
| Red iron oxide (Cl 77491) | 0.288218000 |
| Stearic Acid | 0.259022200 |
| Alumina | 0.231019800 |
| *Chlorella vulgaris* extract | 0.200000000 |
| Glucose | 0.200000000 |
| aluminum hydroxide | 0.187765000 |
| Triethoxycaprylylsilane | 0.174318000 |
| Dimethicone | 0.150000000 |
| Dimethicone/Vinyl dimethicone crosspolymer | 0.150000000 |
| Ethylhexyl hydroxystearate | 0.150000000 |
| Black iron oxide (Cl 77499) | 0.109956000 |
| Pentylene glycol | 0.106060500 |
| Ethylhexylglycerin | 0.100501000 |
| Matricaria flower extract | 0.100000000 |
| Fructooligosaccharide | 0.100000000 |
| Fructose | 0.100000000 |
| Glyceryl Caprylate | 0.100000000 |
| Vinyl dimethicone/Methicone silsesquioxane crosspolymer | 0.100000000 |
| Fragrances | 0.050000000 |
| Octanediol | 0.045454500 |
| Adenosine | 0.040000000 |
| Trisodium ethylenediamine disuccinate | 0.037000000 |
| Palmitoyl tripeptide-5 | 0.005500000 |
| Magnesium chloride | 0.002500000 |
| Tetradecyl aminobutyroylvalylaminobutyric urea trifluoroacetate | 0.002500000 |
| Tocopherol | 0.000600000 |
| *Epilobium placekeri* extract | 0.000075000 |
| Sodium hyaluronate | 0.000010000 |
| Caprylyl glycol | 0.000005000 |
| Citric acid | 0.000005000 |
| Copper tripeptide-1 | 0.000005000 |
| Heptasodium hexacarboxymethyl dipeptide-12 | 0.000005000 |
| Hexapeptide-2 | 0.000001600 |
| Hydroxypropyl trimonium hyaluronate | 0.000001000 |
| Potassium sorbate | 0.000001000 |
| Acetylhexapeptide-8 | 0.000000500 |
| Biotin | 0.000000400 |
| Hydrolyzed hyaluronic acid | 0.000000100 |
| Sodium acetylated hyaluronate | 0.000000100 |
| Palmitoyl pentapeptide-4 | 0.000000050 |
| Hyaluronic acid | 0.000000025 |
| Hydrolyzed sodium hyaluronate | 0.000000010 |
| Sodium hyaluronate crosspolymer | 0.000000010 |
| Potassium hyaluronate | 0.000000002 |
| Sum | 100 |

### Experimental Example 1. Sensory evaluation for improvement of feeling of use on skin

In the case of water-insoluble extracts or water-insoluble substances derived from microorganisms, there is a problem wherein they often have negative feelings such as slippery or sticky sensations when applied to the skin. Therefore, in order to evaluate the feeling of use on the skin for the porous silica impregnated with the *E. keratini* extract of Example 2, a sensory evaluation was performed.

Specifically, a total of 5 evaluators evaluated the freshness, moisture, and adhesion of each sample by using a 5-point scale. The feeling of use on the skin for each sample was scored from 1 to 5, and then the proportion of people who responded with 3 or higher was calculated (1 point: very poor, 2 points: poor, 3 points: average, 4 points: good, 5 points: very good). The results are shown in Table 2 below.

**[Table 2]**

| **Sample** | **Evaluation score (persons)** | | | | | **Averag e score (point)** |
|---|---|---|---|---|---|---|
| | **5 point s** | **4 point s** | **3 point s** | **2 point s** | **1 po int** | |
| Example 1 (rice bran oil extract of *E. keratini*) | 0 | 0 | 2 | 2 | 1 | 2.2 |
| Control group (untreated porous silica) | 3 | 1 | 1 | 0 | 0 | 4.4 |
| Example 2 (Porous silica impregnated with rice bran oil extract of *E. keratini*) | 2 | 2 | 1 | 0 | 0 | 4.2 |

As shown in Table 2, the porous silica of Example 2 impregnated with the rice bran oil extract of *E. keratini* of Example 1 was found to have a better feeling of use than the rice bran oil extract of *E. keratini* of Example 1. In addition, the feeling of use of the untreated porous silica was similar to Example 2. This means that the negative feeling of use of the rice bran oil extract of *E. keratini* was offset by the positive feeling of use of the porous silica. Therefore, it was confirmed that the porous silica impregnated with the rice bran oil extract of *E. keratini* of Example 2 eliminated the sticky or slippery sensation of water-insoluble components derived from microorganisms and provides a refreshing and light feeling, thereby having an excellent effect of improving the feeling of use on the skin.

### Experimental Example 2. Evaluation of anti-aging and wrinkle amelioration efficacy

The effect of the porous silica impregnated with the rice bran oil extract of *E. keratini* of Example 2 on factors related to skin aging and wrinkle production due to ultraviolet (UV) irradiation was analyzed.

Specifically, a human fibroblast cell line (Human dermal fibroblast, Hs68) was distributed at 3.5×10⁵ in a 6-well plate, and then cultured for 24 hours in an incubator in conditions of 37°C and 5 % CO₂. Afterwards, the medium was removed, DPBS was added, and then 12 mJ/cm² of UVB was irradiated or not irradiated. Immediately after UVB irradiation, DPBS was removed and replaced with a medium without FBS, and then the cells were treated with 1 % (w/v) of the porous silica of Example 2 or the untreated porous silica (control group) and further cultured for 24 hours. Afterwards, RNA was isolated from the cells of each sample by using Trizol (RNA iso, DAKARA, Japan), then RNA was quantified at 260 nm with a nanodrop, and then cDNA was synthesized in an amplifier by using 2 µg of each RNA (C1000 Thermal Cycler, Bio-Rad, USA). Real-time polymerase chain reaction was performed on a real-time PCR machine (Step One Plus, Applied Biosystems, USA) by adding SYBR Green (SYBR Green supermix, Applied Biosystems, USA) along with primers and cDNA to the target genes, collagen synthase (COL1A1) and collagenase (MMP-1), using the synthesized cDNA as a template. The expression level of the gene was finally analyzed through correction for the β-actin gene.

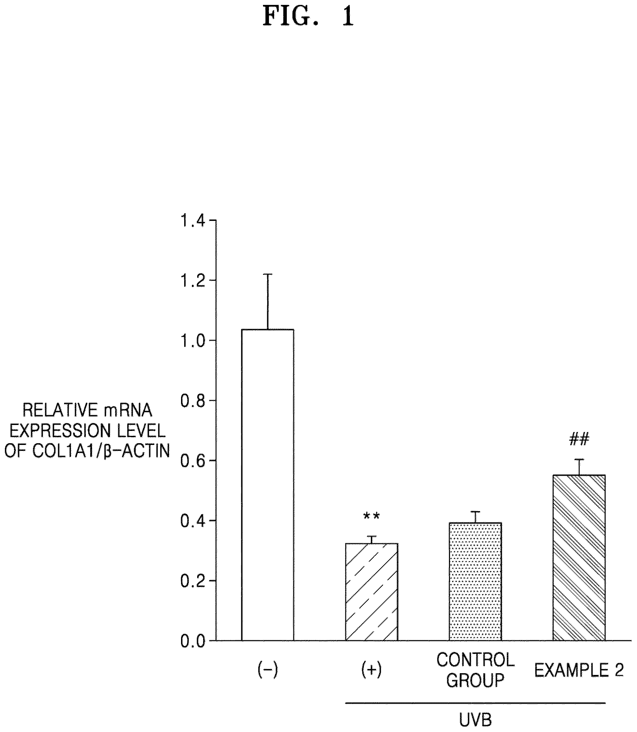

FIG. 1 is results showing the relative expression level of collagen synthase (COL1A1) in fibroblasts after addition of the porous silica of Example 2 or the porous silica of the control group.

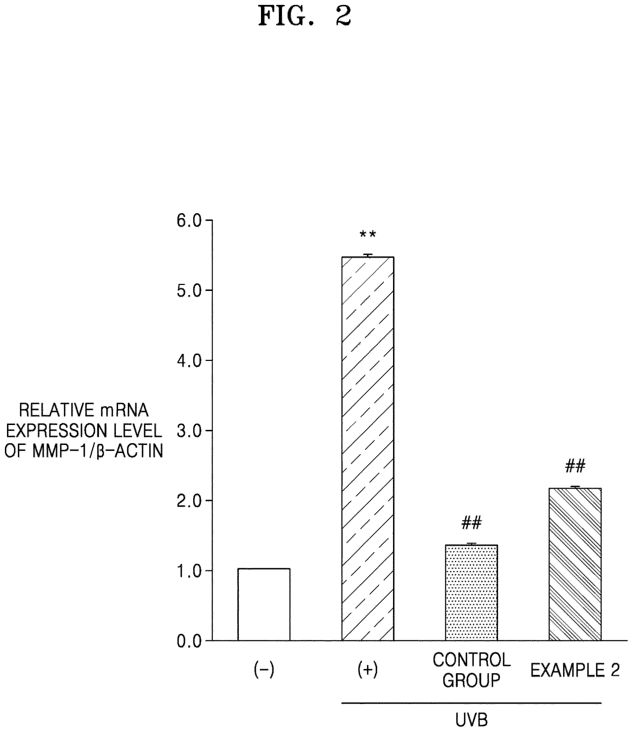

FIG. 2 is results showing the relative expression level of a collagen-degrading enzyme (MMP-1) in fibroblasts after addition of the porous silica of Example 2 or the porous silica of the control group.

As shown in FIGS. 1 and 2, it was confirmed that the porous silica impregnated with the rice bran oil extract of *E. keratini* of Example 2 significantly increased the expression of COL1A1, which had been reduced by ultraviolet rays, and significantly decreased the expression of MMP-1, which had been increased by ultraviolet rays.

These results indicate that the porous silica impregnated with the rice bran oil extract of *E. keratini* has effects of preventing aging (e.g. photoaging) and ameliorating wrinkles.

### Experimental Example 3. Evaluation of skin barrier strengthening and skin moisturizing efficacy

The effect of the porous silica impregnated with the rice bran oil extract of *E. keratini* of Example 2 on skin barrier strengthening and skin moisturizing activity was analyzed.

Specifically, HaCaT cells, a human keratinocyte cell line, were cultured in Dulbecco's modified Eagle's Medium (DMEM medium, Gibco 1210-0038) including 10 % fetal bovine serum (FBS). All cultivations were performed in an incubator at 37°C and 5 % CO₂. The cultured cell line was treated with 1 % (w/v) of the porous silica of Example 2 or the untreated porous silica (control group) and further cultured for 24 hours. The expression levels of HAS3 (hyaluronic acid synthase) and filaggrin were measured in the same manner as in Experimental Example 2, except that primers for HAS3 and filaggrin, which are factors related to skin barrier strengthening and moisturizing, were used.

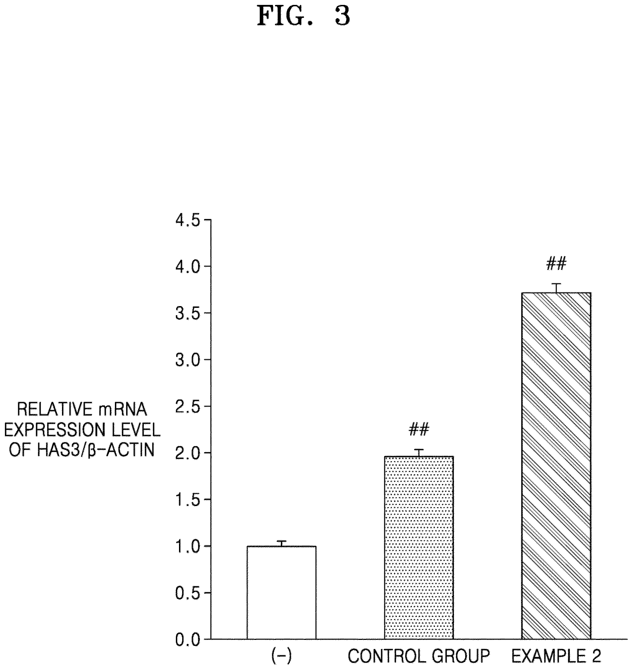

FIG. 3 is results showing the relative expression level of hyaluronic acid synthase (HAS3) in keratinocytes after addition of the porous silica of Example 2 or the control group.

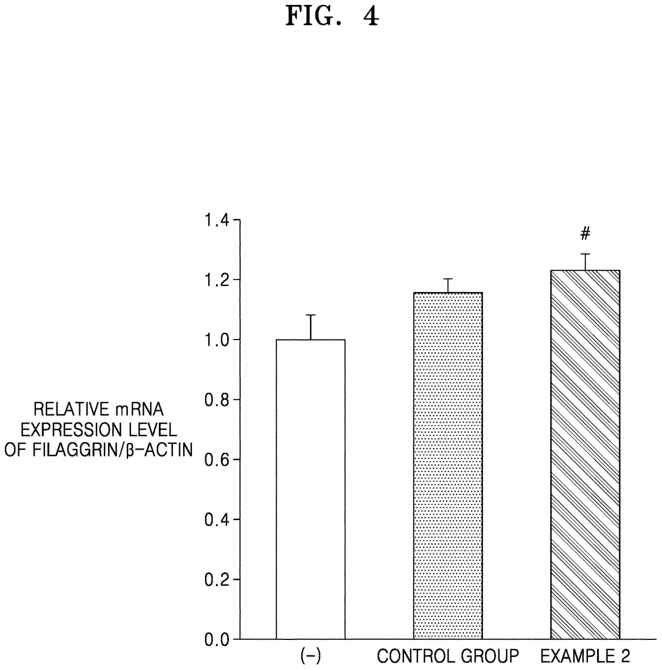

FIG. 4 is results showing the relative expression level of filaggrin in keratinocytes after addition of the porous silica of Example 2 or the control group.

As shown in FIGS. 3 and 4, it was found that the porous silica impregnated with the rice bran oil extract of *E. keratini* of Example 2 significantly increased the expression levels of both HAS3 and filaggrin.

These results mean that the porous silica impregnated with the rice bran oil extract of *E. keratini* has effects of strengthening skin barrier and moisturizing skin.

### Experimental Example 4. Clinical evaluation of skin barrier strengthening efficacy

The effect of the porous silica impregnated with the rice bran oil extract of *E. keratini* of Example 2 on skin barrier was analyzed. The experiment was conducted at the Korea Dermatological Research Institute.

Specifically, the test was performed according to the details below.
1) Selection of research subjects
   : 21 selected research subjects who met the selection criteria and did not fall under the exclusion criteria.
2) Test site
   : Point 1 cm horizontally outward from the lateral canthus
3) How to use
   : Research subjects use the sample on their own face twice a day, morning and evening, for 3 weeks.
4) Evaluation
   : Transepidermal water loss (TEWL) evaluation using a Tewameter

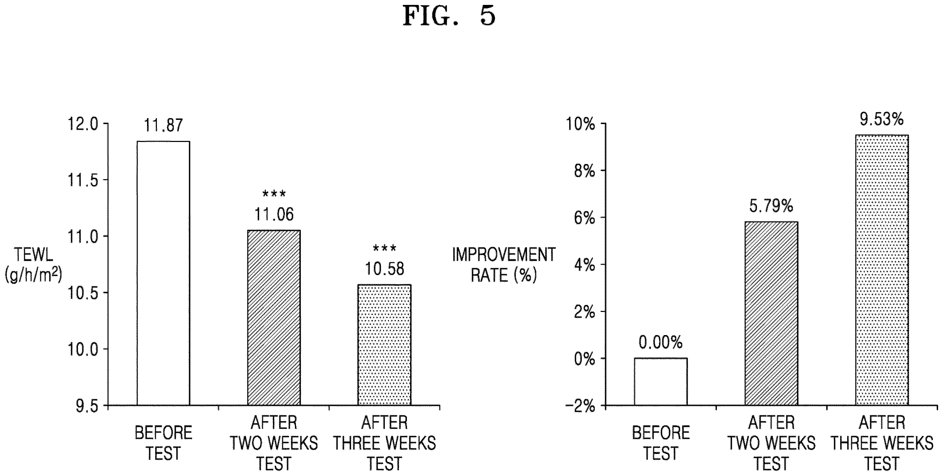

FIG. 5 is the result of evaluating the transepidermal water loss (TEWL) after using the porous silica of Example 2.

As shown in FIG. 5, it was found that the porous silica impregnated with the rice bran oil extract of *E. keratini* of Example 2 reduced the transepidermal water loss (TEWL).

These results indicate that the porous silica impregnated with the rice bran oil extract of *E. keratini* may help strengthen the skin barrier.

### Experimental Example 5. Clinical evaluation of skin moisturizing improvement efficacy

The effect of the porous silica impregnated with the rice bran oil extract of *E. keratini* of Example 2 on skin moisturization was analyzed. The experiment was conducted at the Korea Dermatological Research Institute.

Specifically, the test was performed according to the details below.
1) Selection of research subjects
   : 21 selected research subjects who met the selection criteria and did not fall under the exclusion criteria.
2) Test site
   : Intersection point between the horizontal line at the alare and the ipsilateral mid pupillary line
3) How to use
   : Research subjects use the sample on their own face twice a day, morning and evening, for 3 weeks.
4) Evaluation
   : Skin moisture content evaluation using a Corneometer^{®}

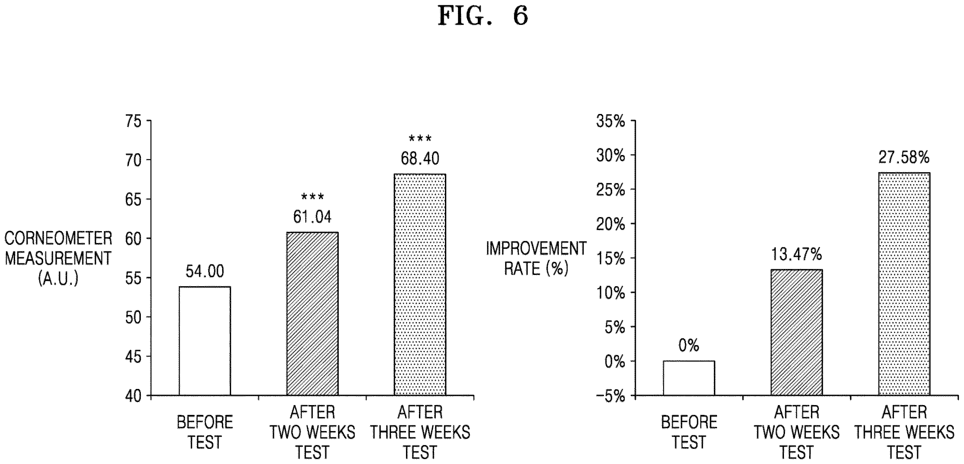

FIG. 6 is the result of evaluating the skin moisture content after using the porous silica of Example 2.

As shown in FIG. 6, it was found that the porous silica impregnated with the rice bran oil extract of *E. keratini* of Example 2 increased the skin moisture content.

These results indicate that the porous silica impregnated with the rice bran oil extract of *E. keratini* may help improve the skin moisture content (moisturizing power).

### Experimental Example 6. Clinical evaluation of skin elasticity improvement efficacy

The effect of the porous silica impregnated with the rice bran oil extract of *E. keratini* of Example 2 on epidermal elasticity was analyzed. The experiment was conducted at the Korea Dermatological Research Institute.

Specifically, the test was performed according to the details below.
1) Selection of research subjects
   : 21 selected research subjects who met the selection criteria and did not fall under the exclusion criteria.
2) Test site
   : 1/2 point of the imaginary line connecting the lip commissure and the ipsilateral tragus
3) How to use
   : Research subjects use the sample on their own face twice a day, morning and evening, for 3 weeks.
4) Evaluation
   : Epidermal elasticity evaluation using a Cutometer^{®} MPA 580 (2mm probe)

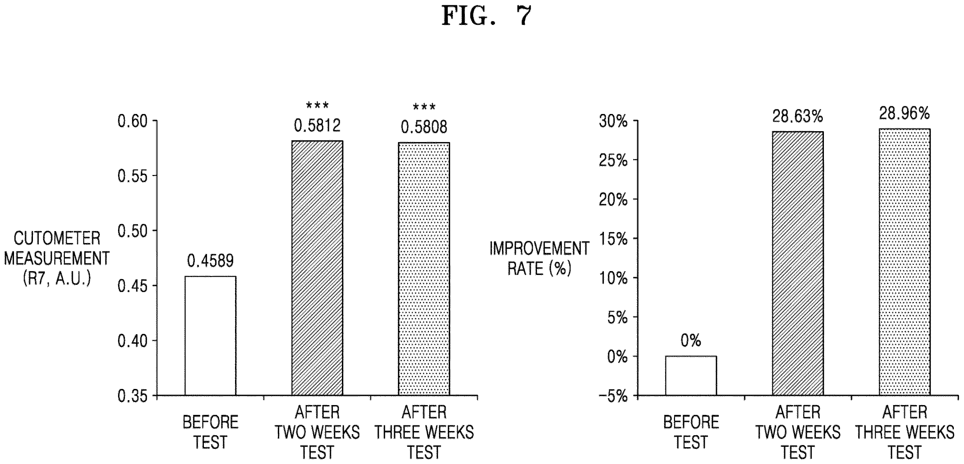

FIG. 7 is the result of evaluating the epidermal elasticity after using the porous silica of Example 2.

As shown in FIG. 7, it was found that the porous silica impregnated with the rice bran oil extract of *E. keratini* of Example 2 increased the epidermal elasticity.

These results indicate that the porous silica impregnated with the rice bran oil extract of *E. keratini* may help enhance skin elasticity.

### Experimental Example 7. Clinical evaluation of exfoliation improvement efficacy

The effect of the porous silica impregnated with the rice bran oil extract of *E. keratini* of Example 2 on dead skin cells was analyzed. The experiment was conducted at the Korea Dermatological Research Institute.

Specifically, the test was performed according to the details below.
1) Selection of research subjects
   : 21 selected research subjects who met the selection criteria and did not fall under the exclusion criteria.
2) Test site
   : Intersection point between the horizontal line at the alare and the ipsilateral mid pupillary line
3) How to use
   : Research subjects use the sample on their own face twice a day, morning and evening, for 3 weeks.
4) Evaluation
   : Desquamation index evaluation using Corneofix and Visioscan

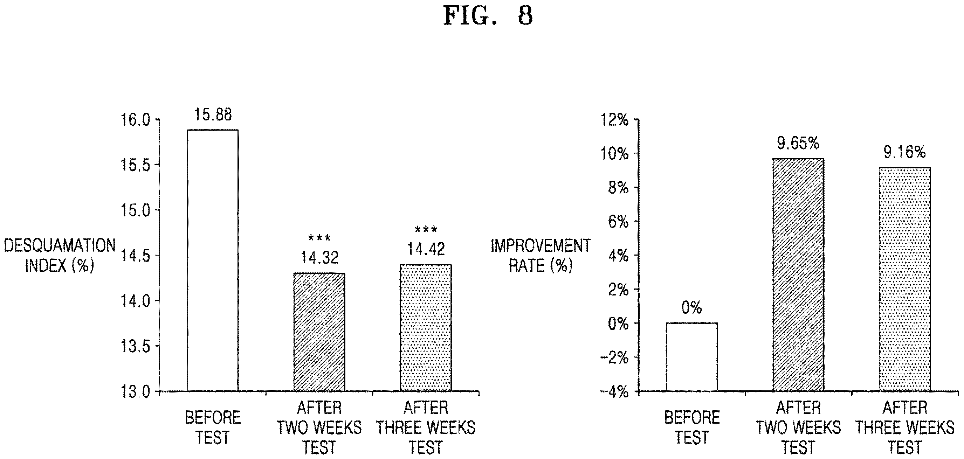

FIG. 8 is results of evaluating the skin desquamation index after using the porous silica of Example 2.

As shown in FIG. 8, it was found that the porous silica impregnated with the rice bran oil extract of *E. keratini* of Example 2 reduced the desquamation index.

These results indicate that the porous silica impregnated with the rice bran oil extract of *E. keratini* may help ameliorate dead skin cells.

### Experimental Example 8. Clinical evaluation of skin gloss improvement efficacy

The effect of the porous silica impregnated with the rice bran oil extract of *E. keratini* of Example 2 on improving skin gloss was analyzed. The experiment was conducted at the Korea Dermatological Research Institute.

Specifically, the test was performed according to the details below.
1) Selection of research subjects
   : 21 selected research subjects who met the selection criteria and did not fall under the exclusion criteria.
2) Test site
   : The most prominent point of the imaginary vertical line passing through the lateral canthus and the zygomatic bone.
3) How to use
   : Research subjects use the sample on their own face twice a day, morning and evening, for 3 weeks.
4) Evaluation
   : Skin gloss value evaluation using a Glossymeter

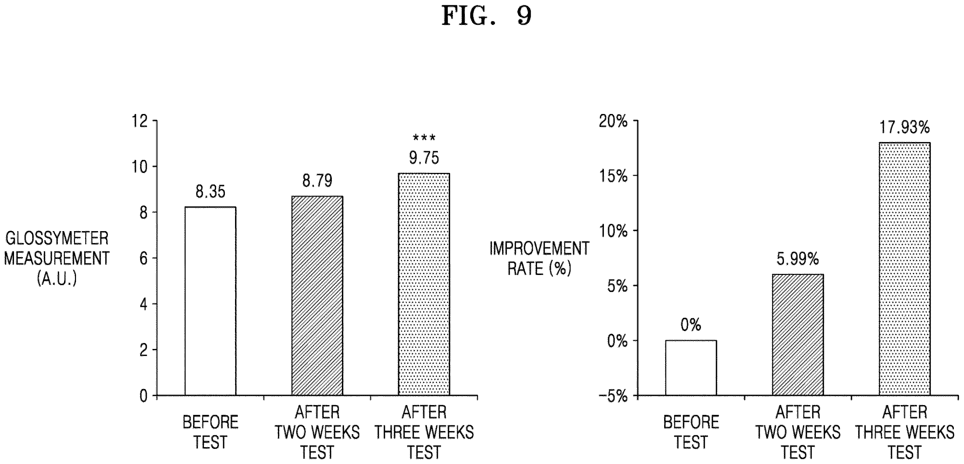

FIG. 9 is results of evaluating the skin gloss value after using the porous silica of Example 2.

As shown in FIG. 9, it was found that the porous silica impregnated with the rice bran oil extract of *E. keratini* of Example 2 increased the total skin gloss value.

These results indicate that the porous silica impregnated with the rice bran oil extract of *E. keratini* may help improve skin gloss.

### Experimental Example 9. Clinical evaluation of skin transparency improvement efficacy

The effect of the porous silica impregnated with the rice bran oil extract of *E. keratini* of Example 2 on skin transparency was analyzed. The experiment was conducted at the Korea Dermatological Research Institute.

Specifically, the test was performed according to the details below.
1) Selection of research subjects
   : 21 selected research subjects who met the selection criteria and did not fall under the exclusion criteria.
2) Test site
   : 1/2 point of the imaginary line connecting the lip commissure and the ipsilateral tragus
3) How to use
   : Research subjects use the sample on their own face twice a day, morning and evening, for 3 weeks.
4) Evaluation
   : Skin transparency evaluation using a Translucency meter

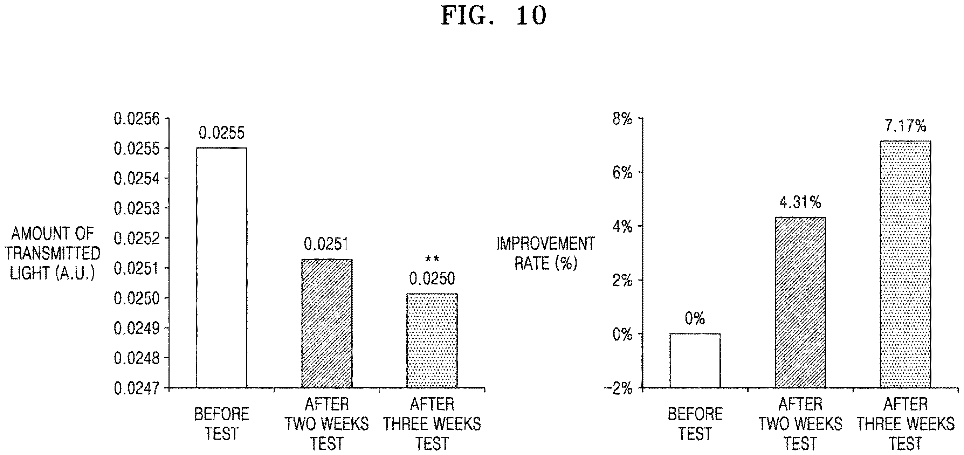

FIG. 10 is results of evaluating skin transparency after using the porous silica of Example 2.

As shown in FIG. 10, it was found that the porous silica impregnated with the rice bran oil extract of *E. keratini* of Example 2 reduced the rate of decrease in an amount of transmitted light (alpha value).

These results indicate that the porous silica impregnated with the rice bran oil extract of *E. keratini* may help improve skin transparency.

### Experimental Example 10. Clinical evaluation of skin tone improvement efficacy

The effect of the porous silica impregnated with the rice bran oil extract of *E. keratini* of Example 2 on skin tone was analyzed. The experiment was conducted at the Korea Dermatological Research Institute.

Specifically, the test was performed according to the details below.
1) Selection of research subjects
   : 21 selected research subjects who met the selection criteria and did not fall under the exclusion criteria.
2) Test site
   : Intersection point between the horizontal line at the alare and the ipsilateral mid pupillary line
3) How to use
   : Research subjects use the sample on their own face twice a day, morning and evening, for 3 weeks.
4) Evaluation
   : Skin brightness evaluation using a Chromameter

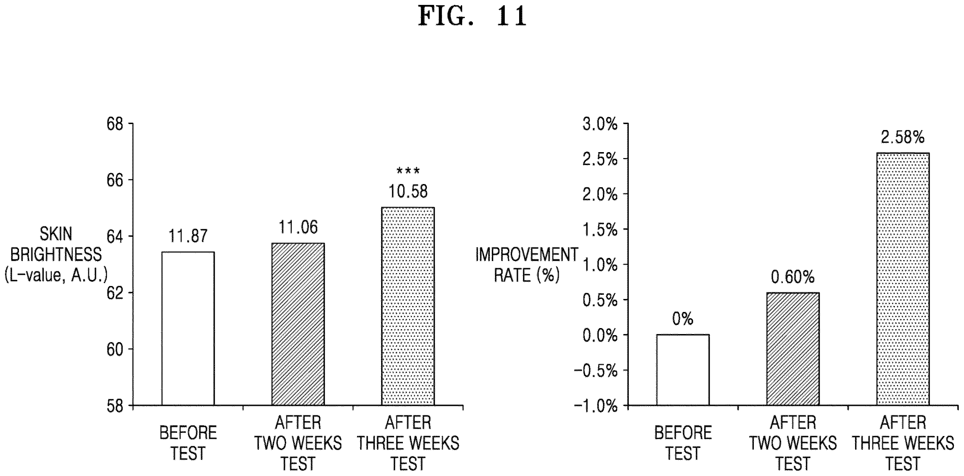

FIG. 11 is results of evaluating skin brightness after using the porous silica of Example 2.

As shown in FIG. 11, it was found that the porous silica impregnated with the rice bran oil extract of *E. keratini* of Example 2 increased skin brightness (L-value).

These results indicate that the porous silica impregnated with the rice bran oil extract of *E. keratini* can help improve skin tone.

In summary, it was confrimed that the porous silica impregnated with the rice bran oil extract of *E. keratini* had an excellent effect of improving the feeling of use on the skin by eliminating the sticky or slippery sensation of water-insoluble components derived from microorganisms; and had effects of preventing skin aging and ameliorating skin wrinkles (e.g. increased COL1A1 expression and decreased MMP-1 expression), strengthening skin barrier and moisturizing skin (e.g. increased HAS and filaggrin expression, reduced transepidermal water loss, and increased skin moisture content), enhancing skin elasticity, ameliorating dead skin cells, improving skin gloss, improving skin transparency, and improving skin tone. Therefore, the porous silica impregnated with the rice bran oil extract of *E. keratini* may be applied in a functional cosmetic composition.

The above description is merely an illustrative explanation of the technical idea of the present disclosure, and various modifications and variations will be possible to those skilled in the art without departing from the essential characteristics of the present disclosure.

## Claims

1. Porous silica impregnated with an extract of an *Epidermidibacterium keratini* strain.

2. The porous silica of claim 1, wherein an extraction solvent for the extract is oil.

3. The porous silica of claim 1, wherein the oil is one or more selected from the group consisting of rice bran oil, MCT oil, soybean oil, sunflower seed oil, jojoba oil, mineral oil, squalane, caprylic/capric triglyceride, cetyl ethylhexanoate, dodecane, isododecane, and hydrogenated polydecene.

4. The porous silica of claim 1, wherein the porous silica consists of silicon dioxide.

5. The porous silica of claim 1, wherein a surface of the porous silica is hydrophobically coated.

6. The porous silica of claim 1, wherein a surface of the porous silica is coated with methicone, dimethicone, or copolymers or mixtures thereof.

7. A cosmetic composition comprising the porous silica of any one of claims 1 to 6.

8. The cosmetic composition of claim 7, wherein the cosmetic composition is for preventing skin aging, ameliorating skin wrinkles, strengthening skin barrier, moisturizing skin, enhancing skin elasticity, ameliorating dead skin cells, improving skin gloss, improving skin transparency, or improving skin tone.

9. A method of producing the cosmetic composition of claim 7, the method comprising:
producing an extract of an *Epidermidibacterium keratini* strain by using oil as an extraction solvent;
impregnating the extract into porous silica; and
producing a cosmetic composition comprising the porous silica impregnated with the extract.

10. The method of claim 9, wherein the porous silica is hydrophobically coated.
